# EUROPÄISCHE PATENTSCHRIFT

(11) **EP 2 767 596 B1**
(45) Veröffentlichungstag und Bekanntmachung des Hinweises auf die Patenterteilung: **10.02.2016**
(21) Anmeldenummer: 13155557.5
(22) Anmeldetag: 16.02.2013
(51) Int. Cl.: C07H 3/04, C07H 1/00, C13K 5/00

(54) **Verfahren zur Steigerung der Ausbeute bei der Lactoseherstellung (III)**
Process for increasing yield in the production of lactose (III)
Procédé d'augmentation du rendement pour la fabrication de lactose (III)

(43) Veröffentlichungstag der Anmeldung: 20.08.2014
(73) Patentinhaber: DMK Deutsches Milchkontor GmbH, 27404 Zeven (DE)
(72) Erfinder: Döring, Sven-Rainer, 27404 Zeven (DE)
(74) Vertreter: Fabry, Bernd

(56) Entgegenhaltungen:
- EP-A1- 0 052 541
- WO-A1-2012/047122
- SHIN YEE WONG ET AL: "Designing a lactose crystallization process based on dynamic metastable limit", JOURNAL OF FOOD ENGINEERING, BARKING, ESSEX, GB, Bd. 111, Nr. 4, 1. März 2012 (2012-03-01), Seiten 642-654, XP028417175, ISSN: 0260-8774, DOI: 10.1016/J.JFOODENG.2012.03.003 [gefunden am 2012-03-20]

## Beschreibung

### GEBIET DER ERFINDUNG

Die Erfindung befindet sich auf dem Gebiet der Molkereiindustrie und betrifft ein verbessertes Verfahren zur Herstellung von Lactose.

### STAND DER TECHNIK

Lactose gehört zur Gruppe der Disaccharide und besteht aus den beiden Molekülen D-Galactose und D-Glucose, die über eine β-1,4-glycosidische Bindung verbunden sind.

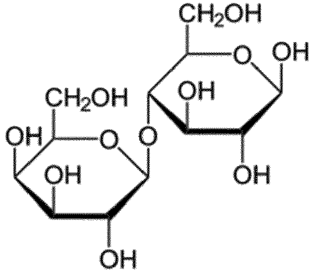

Lactose ist eine kristalline, farblose Substanz mit süßem Geschmack; die Süßkraft liegt je nach Konzentration zwischen 25 und 60 % der von Saccharose. Lactose stellt einen wichtigen Bestandteil der Milch dar und verfügt über eine Vielzahl von ernährungsphysiologischen Vorteilen. So dient es dem Metabolismus als Energiequelle, unterstützt die Calcium-resorption, hemmt die Bildung von Fäulnisbakterien im Darm und wirkt zumindest in größeren Mengen eingenommen, abführend. In der Lebensmitteltechnik wird es vor allem zur Herstellung von Milchsäure und als Texturiermittel für Tiefkühlprodukte eingesetzt. Da es Lebensmittel einen cremigen Geschmack verleiht, stellt es ein weit verbreitetes Additiv dar.

Lactose stellt ein Nebenprodukt bei der Herstellung von Proteinpulvern dar. Dabei wird üblicherweise Molke durch Ultrafiltration von Proteinen befreit, die anschließend einer Sprühtrocknung unterworfen werden.

Ein heute übliches Verfahren zur Herstellung von Lactose wird beispielsweise in der internationalen Patentanmeldung WO 2002 050089 A1 (Food Science Australia) beschrieben. Hierzu wird im ersten Verfahrensschritt das bei der Ultrafiltration der Molke gewonnene Permeat durch Umkehrosmose (RO) oder Nanofiltration (NF) aufkonzentriert. Anschließend wird das so gewonnene Konzentrat in zwei Schritten demineralisiert, d.h. zunächst mit einem Erdalkalisalz, üblicherweise einer wässrigen Calciumchloridlösung versetzt, wobei die Mineralien als Calciumphosphat gefällt werden. In der zweiten Fällungsstufe wird die Löslichkeit der Calciumsalze durch Zugabe von niederen Alkoholen weiter herabgesetzt und weiteres Phosphat gefällt. Die Abtrennung der Salze erfolgt dann mit Hilfe von geeigneten Filtrationsvorrichtungen, wie z.B. Membranen, Separatoren oder dergleichen. Anschließend wird die gereinigte Lactoselösung einer Vakuumdestillation unterworfen und auf einen Feststoffgehalt von etwa 65 Gew.-% eingestellt.

Auch das Patent US 4,202,909 beschreibt ein Verfahren zur Gewinnung von Lactose, bei dem man zunächst Molke einer Ultrafiltration unterwirft, das UF-Permeat einer Demineralisierung unterwirft, das Permeat aufkonzentriert und die Lactose dann aus der Mutterlauge abscheidet. Insbesondere wird beschrieben, dass die Mutterlauge dann noch einmal demineralisiert und aufgearbeitet werden kann, um eine weitere Menge Lactose zu gewinnen. Ähnlichen Inhalts ist auch die GB 1575089 B **bei der** in Beispiel 1 ein Verfahren zur Gewinnung von Lactose beschrieben wird, bei dem man zunächst Molke einer Ultrafiltration unterwirft, das UF-Permeat einer Demineralisierung unterwirft, das Permeat aufkonzentriert und die Lactose dann aus der Mutterlauge abscheidet.

Verfahren zur Herstellung von kristalliner Lactose wurden auch in EP0052541 A1, WO2012/047122 A1 und von S.Wong in J. of Food Engineering 111 (2012), 642-654 beschrieben.

Üblich nach den Verfahren des Stands der Technik ist, dass die Mutterlauge, die etwa 90 Gew.-% Lactose in der Trockenmasse enthält, nach dem Eindampfer in den Kristallisationstank gefördert wird. Dieser wird zum Befüllen geheizt, um die Lactose in Lösung zu halten. Oberhalb von 93,5 °C liegt reine beta-Lactose vor. Beim Abkühlen wandelt sich diese jedoch quantitativ in alpha-Lactose um. Allerdings handelt es sich hier nicht um einen scharfen Phasenübergang, denn zunächst wird eine metastabile Phase durchlaufen, aus der sich dann die alpha-Lactosekristalle abscheiden. Beim weiteren Abkühlen folgt man in der Regel der Sättigungskurve, damit die Kristalle ausreichend Zeit haben zu wachsen.

Nach dem Beginn des Kristallisationsprozesses wird auf eine Temperatur von 30 bis 40 °C abgekühlt, die Lauge gegebenenfalls für 1 bis 3 h bei dieser Temperatur ruhen gelassen und dann weiter auf 10 °C abgekühlt. Die Gesamte Kühlzeit beträgt etwa 20 h.

Problematisch ist, dass bei diesem Kühlverlauf immer wieder Phasen metabstabilen Zustandes durchschritten werden, bei denen sich erneut frische Kristallkeime bilden können, die jedoch wenig Zeit zum Wachsen haben und sehr klein bleiben. Da bei der Abscheidung der alpha-Lactose Kristalle im Dekanter die frisch gebildeten kleinen Kristalle mit einem Durchmesser von weniger als 80 µm nicht erfasst werden, gehen etwa 17 % der Lactose verloren.

Durch das Rühren kommt es im Dekanter zudem zu Kristallbruch, wobei wiederum Material entsteht, dass wegen seines zu geringen Teilchendurchmessers nicht abgeschieden werden kann. Auf diese Weise gehen weitere 20 % der Lactose verloren, so dass die Mutterlauge schließlich noch 35 bis 40 % Lactose enthält, die mit hohem Aufwand wieder aufgearbeitet werden muss, um in der Gesamtbetrachtung des Verfahrens zu einer akzeptablen Ausbeute zu gelangen.

Es liegt auf der Hand, dass jede Verfahrensverbesserung, die dazu führt, dass sich die Restmenge an Lactose in der Mutterlauge verringert, einen erheblichen Einfluss auf die Ökonomie des Verfahrens hätte. Somit hat die Aufgabe der vorliegenden Erfindung darin bestanden, bestehende Verfahren zur Lactoseherstellung dahingehend zu verbessern und die Restmenge an alpha-Lactose, die nach der Kristallisation mit der Mutterlauge verloren geht, auf maximal 10 Gew.-% zu begrenzen.

### BESCHREIBUNG DER ERFIUNDUNG

Gegenstand der Erfindung ist ein Verfahren zur Verbesserung der Ausbeute bei der Herstellung von kristalliner alpha-Lactose, bei dem man
(a) eine wässrige Lactoselösung auf eine Temperatur von etwa 62 bis 67 °C einstellt,
(b) die Lösung dann bis auf etwa 20 bis 30 °C abkühlt,
(c) die Lösung bei dieser Temperatur für 0 bis 5 h hält,
(d) anschließend die Lösung wieder bis auf etwa 30 bis 35 °C erwärmt,
(e) die Lösung bei dieser Temperatur für 0 bis 5 h hält,
(f) danach die Lösung bis auf etwa 10 °C abkühlt und
(g) anschließend die ausgefallenen alpha-Lactosekristalle von der zweiten Mutterlauge abtrennt,
(h) die Mutterlauge mit einer solchen Menge an Kohlenhydratverbindungen aus gewählt aus der Gruppe, die gebildet wird von L-Glycerinaldehyd, Dihydroxyaceton; D-Erythrose, D-Threose; D-Ribose, D- und L-Xylulose; D-Glucose, D-Mannose, D-Gulose, D-Idose, D-Galaktose, D-Glucronsäure, D-Galacturonsäure, N-Acetyl-D-glucosamin, D-Glucosamin, D-Fructose, L-Rhamnose; Cellobiose, Gentiobiose, Isomaltose, Isomaltulose, Lactulose, Laminaribiose, Maltose, Maltulose, Melibiose, Neohesperidose, Neotrehalose, Nigerose, Rutinose, Sophorose, Saccharose und Trehalose sowie deren Gemischen versetzt, dass das Löslichkeitsprodukt der noch in Lösung befindlichen Restmenge an Lactose überschritten wird,
(i) die zweite Menge ausgefallener alpha-Lactosekristalle von der Mutterlauge abtrennt, trocknet und
(j) die beiden Mengen an alpha-Lactosekristallen vereinigt.

Überraschenderweise wurde gefunden, dass durch zwischenzeitliches Aufheizen der Lactoselösung kleine Kristalle und Kristallabrieb wieder in Lösung gehen und damit wieder zur Verfügung stehen, um auf großen Kristallen aufzuwachsen. Auf diese Weise kann der Verlust an Lactose durch Kristallbruch und mangelhaftes Dekantieren etwa halbiert werden, so dass die nach dem Dekantieren zurückbleibende Mutterlauge nur noch einen Lactosegehalt von maximal 20 Gew.-% aufweist. Durch Zugabe von bis zu 5 Gew.-% eines Zuckers, speziell von Glucose, kann das Löslichkeitsprodukt der Lactose verschoben werden, so dass in der Mutterlauge nur noch etwa 5 bis 10 Gew.-% Lactose verbleiben.

### Lactoselösungen

Lactoselösungen, die im Sinne des erfindungsgemäßen Verfahrens als Ausgangsstoffe für die Gewinnung der alpha-Lactosekristalle in Betracht kommen, werden in der Regel ausgehend von Molke gewonnen. Dazu wird Molke zunächst in eine proteinreiche und eine lactosereiche Fraktion aufgespalten. Das bevorzugte Trennverfahren stellt hierfür die Ultrafiltration (UF) dar, bei der das UF Retentat zur Gewinnung von Proteinen weiterverarbeitet und das UF Permeat für die Gewinnung der Lactose verwendet wird. Typischerweise enthält das UF Retentat etwa 20 Gew.-% Trockenmasse und davon etwa 2 Gew.-% Lactose, der Aschegehalt liegt bei etwa 1 Gew.-%. Das UF Permeat, das zur Herstellung der Lactose weiterverarbeitet wird, weist hingegen eine Trockenmasse von etwa 4,5 bis 5,5 Gew.-% auf, wobei der Gehalt an Lactose bei ca. 4,1 bis 4,6 und der Aschegehalt bei etwa 0,3 bis 0,5 liegt.

Als optionaler, jedoch im Allgemeinen bevorzugter Schritt, wird das UF Permeat aufkonzentriert, wobei man eine Trockenmasse von etwa 10 bis 30 Gew.-% - entsprechend 10 bis 30 °Brix - einstellt. Dies erfolgt vorzugsweise entweder durch Umkehrosmose (RO) oder Nanofiltration (NF).

Das UF Permeat weist - gegebenenfalls nach Aufkonzentrieren - einen Gehalt an Mineralien in der Größenordnung von 1 bis 2 Gew.-% auf. Um die Spezifikation, die bei unter 0,3 Gew.-% liegt, erreichen zu können, werden die Lösungen durch Zugabe von Basen zunächst auf einen annähernd neutralen pH-Wert im Bereich von 6 bis 8 eingestellt und die Mineralien, die im Wesentlichen lösliche Phosphate darstellen mit einer solchen Menge einer Lösung eines wasserlöslichen Calciumsalzes versetzt, dass schwerlösliche Calciumsalze gefällt werden. Zur Einstellung des pH-Wertes und zur Fällung werden NaOH, eine wässrige Zubereitung aus Calciumchlorid und Alkalihydroxid oder Calciumhydroxid einsetzt. Grundsätzlich können zur Einstellung des pH-Wertes auch andere Alkali- oder Erdalkalibasen, wie z.B.-KOH eingesetzt werden. Auch die Natur des Fällungssalzes ist an sich unkritisch, es lassen sich beispielsweise Bariumsalze fällen. Die Verwendung von Calciumsalzen hat indes den Vorteil, dass das Fällungsmittel kostengünstig und die Salze ein sehr niedriges Löslichkeitsprodukt haben, die Fällung also im Wesentlichen vollständig ist. Auch ohne Zugabe von Fällungsmitteln erfolgt die Demineralisierung in Rührkesseln, wobei es sich als vorteilhaft erwiesen hat, eine Temperatur im Bereich von etwa 50 bis 90 und vorzugsweise von etwa 80 °C einzustellen. Die Fällungszeit liegt typischerweise bei etwa 20 bis 120 und vorzugsweise etwa 30 bis 45 min, wobei diese Angaben nur als Anhaltspunkte zu verstehen sind, da niedrigere Temperaturen längere Reaktionszeiten erfordern und umgekehrt. Nach der Fällung werden die Salze abgetrennt, beispielsweise in Separatoren, welche das größere spezifische Gewicht der gefällten Partikel ausnutzen. Es ist aber ebenso möglich, die Abtrennung beispielsweise durch Membranfilter im Rahmen einer weiteren Ultrafiltration im Bereich 5 bis 150 kDa, vorzugsweise 10 bis 50 kDa vorzunehmen.

Der gereinigte Strom enthält jetzt typischerweise etwa 15 bis 20, vorzugsweise etwa 17,5 Gew.-% Lactose, während der Aschegehalt bereits auf etwa 0,8 vermindert worden ist. Falls gewünscht oder erforderlich, kann sich nun eine zweite Demineralisierungsstufe anschließen, bei der man dem vorgereinigten Strom eine Menge an niederen Alkoholen, insbesondere an Ethanol zusetzt, um das Löslichkeitsprodukt der noch enthaltenen Calciumsalze weiter herabzusetzen. Auf diese Weise kann im Bedarfsfall eine weitere Menge an Salzen gefällt und wie oben beschrieben abgetrennt werden.

Als zweiter optionaler, jedoch im Allgemeinen ebenfalls bevorzugter Schritt, wird der demineralisierte lactosereiche Strom nach Verlassen der Filtrationsanlage ein weiteres Mal aufkonzentriert, wobei man einen Feststoffgehalt - im Wesentlichen identisch mit dem Gehalt an Lactose - von etwa 50 bis 70 Gew.-% - entsprechend 40 bis 50 °Brix - einstellt. Dies erfolgt vorzugsweise durch eine Vakuumeindampfung, bei der das Produkt auf vorzugsweise etwa 65 Gew.-% entwässert und gegebenenfalls auch Alkohol aus der Demineralisierungsstufe abgetrennt wird. Die so erhaltenen wässrigen Lactoselösungen können nun in die Kristallisationsstufe eingesetzt werden.

### Kristallisationsverfahren

Im ersten Schritt des Verfahrens wird eine Lactoselösung, die wie oben beschrieben erhältlich ist und etwa 60 bis 95 und vorzugsweise etwa 85 bis 90 Gew.-% Lactose in der Trockenmasse aufweist, in einen vorgeheizten Kristallisationstank gepumpt. Dies kann anlagenspezifisch kontinuierlich oder batchweise erfolgen. Die Mutterlauge kann vor dem Befüllen des Tanks auf eine Temperatur oberhalb von 93,5 °C eingestellt werden, um die Bildung von alpha-Lactose zu verhindern; dies ist jedoch nicht zwingend. Es können auch Mutterlaugen eingesetzt werden, bei denen die Umwandlung von beta- in alpha-Lactose schon begonnen hat. Im Tank wird die Lösung dann auf 62 bis 67 und vorzugsweise etwa 63 bis 65 °C abgekühlt. Dazu reicht es aus, die heiße Lactoselösung sich einfach an die Starttemperatur des Kristallisationstanks anpassen zu lassen, was innerhalb von 1 bis 2 h üblicherweise der Fall ist.

Als dann wird die Lactoselösung kontinuierlich mit einer Geschwindigkeit von etwa 1 bis 5 °K/h bis auf 20 bis 30 und vorzugsweise etwa 23 bis 26 °C abgekühlt und dort für etwa 0 bis 5 und vorzugsweise etwa 1 bis 3 h gehalten. Bei diesem Temperaturverlauf werden immer wieder metastabile Phasen durchschritten in denen es je nach Temperaturrichtung zur Keimbildung oder zum Auflösen der Keime kommt. Es entstehen letztlich aber wieder neue Kristallkeime, die bei weiterer Abkühlung kaum Chancen haben eine hinreichende Größe zu erreichen, um im Dekanter abgetrennt werden zu können. Deshalb wird die Lösung im nächsten Schritt wieder leicht erwärmt, nämlich bis auf etwa 30 bis 45 und vorzugsweise etwa 32 bis 42°C. Bei dieser Temperatur wird die Lösung wiederum etwa 0,5 bis 5 und insbesondere etwa 1 bis 3 h belassen. Die Erwärmung kann grundsätzlich so rasch wie möglich erfolgen, beispielsweise aber wieder mit einer Geschwindigkeit von etwa 1 bis 5 °K/h.

Durch diese Maßnahmen werden die Kristallkeime wieder in Lösung gebracht und haben nun die Möglichkeit auf den größeren Kristallen aufzuwachsen. Dazu wird die Lösung nun mit einer Geschwindigkeit von etwa 1 bis 3 °K/h auf etwa 10 °C, vorzugsweise 5 bis 10 °C abgekühlt und bei dieser Temperatur für etwa 12 bis 15 h gehalten, so dass die alpha-Lactosekristalle hinreichend Zeit haben sich abzuscheiden.

Insgesamt ist für das Durchlaufen des gesamten Kühlprozesses eine Zeit von etwa 18 bis 24 und vorzugsweise etwa 20 h anzusetzen. Anschließend werden die Lactosekristalle von der Mutterlauge getrennt, was vorzugsweise mit Hilfe von Dekantern die nach dem Zentrifugalprinzip arbeiten erfolgt. Grundsätzlich ist aber auch jedes andere Bauteil, mit dessen Hilfe eine Fest/Flüssig-Trennung möglich ist, für diesen Schritt geeignet. Dazu gehören beispielsweise auch Separatoren, die mit Membranen arbeiten. Die Lactosekristalle, an denen noch Mutterlauge anhaftet, werden im Anschluss schonend getrocknet, wozu sich insbesondere Bandtrockner als geeignet erwiesen haben.

### Zugabe der Kohlenhydrate

Als Kohlenhydrate, die geeignet sind, das Löslichkeitsprodukt der Lactose signifikant herabzusetzen, kommen insbesondere Mono- und/oder Disaccharide von Kohlenhydraten und dabei sowohl Aldosen als auch Ketosen in Betracht. Typische Beispiele umfassen
- Triosen, wie z.B. D- und L-Glycerinaldehyd oder Dihydroxyaceton;
- Tetrosen, wie z.B. D-Erythrose oder D-Threose;
- Pentosen, wie z.B. D-Ribose, D- und L-Xylulose;
- Hexosen, wie z.B. D-Glucose, D-Mannose, D-Gulose, D-Idose, D-Galaktose, D-Glucronsäure, D-Galacturonsäure, N-Acetyl-D-glucosamin, D-Glucosamin, D-Fructose, und L-Rhamnose;
- Glucose-basierte Disaccharide, wie z.B. Cellobiose, Gentiobiose, Isomaltose, Isomaltulose, Lactulose, Laminaribiose, Maltose, Maltulose, Melibiose, Neohesperidose, Neotrehalose, Nigerose, Rutinose, Sophorose, Saccharose und Trehalose.

Neben D-Glucose sind vor allem auch glucosereiche Kohlenhydratfraktionen besonders bevorzugt, da sie leicht zugänglich und kostengünstig sind und dabei auch noch zu einer akzeptablen Absenkung des Löslichkeitsproduktes führen. Aus Kostengründen und wegen der leichten Verfügbarkeit kann man besonders bevorzugt Rüben- oder Kornstärkesirup einsetzen.

Die Kohlenhydrate können den Lactosemutterlaugen in Mengen von bis zu etwa 5 Gew.-% zugesetzt werden. Vorzugsweise beträgt die Zusatzmenge etwa 0,1 bis 3 und insbesondere etwa 1 bis 2 Gew.-%.

### GEWERBLICHE ANWENDBARKEIT

Ein weiterer Gegenstand der vorliegenden Patentanmeldung betrifft eine Mutterlauge mit einem Gehalt an alpha-Lactose von etwa 15 bis 20 Gew.-%, die nach dem erfindungsgemäßen Verfahren erhältlich ist.

Ebenfalls von der Erfindung umschlossen ist ein Verfahren zur Herstellung von Lactose, bei dem man
(i) Molke einem Trennverfahren unterwirft, bei dem eine proteinreiche und eine lactosereiche Fraktion erhalten wird,
(ii) die lactosereiche Fraktion gegebenenfalls nach Aufkonzentrierung Hauptstrom) einer Demineralisierung unterwirft, bei der schwerlösliche Salze gefällt werden,
(iii) den demineralisierten Rückstand gegebenenfalls nach weiterer Aufkonzentrierung abkühlt, bis Lactose kristallin ausfällt,
(iv) die Lactosekristalle von der Mutterlauge abtrennt und entwässert,
welches sich dadurch auszeichnet, dass man
(a) die erste Mutterlauge auf eine Temperatur von etwa 62 bis 67 °C einstellt,
(b) die Lösung dann bis auf etwa 20 bis 30 °C abkühlt,
(c) die Lösung bei dieser Temperatur für 0 bis 5 h hält,
(d) anschließend die Lösung wieder bis auf etwa 30 bis 35 °C erwärmt,
(e) die Lösung bei dieser Temperatur für 0 bis 5 h hält,
(f) danach die Lösung bis auf etwa 10 °C abkühlt und
(g) anschließend die ausgefallenen alpha-Lactosekristalle von der zweiten Mutterlauge abtrennt,
(h) die Mutterlauge mit einer solchen Menge an Kohlenhydratverbindungen aus gewählt aus der Gruppe, die gebildet wird von L-Glycerinaldehyd, Dihydroxyaceton; D-Erythrose, D-Threose; D-Ribose, D- und L-Xylulose; D-Glucose, D-Mannose, D-Gulose, D-Idose, D-Galaktose, D-Glucronsäure, D-Galacturonsäure, N-Acetyl-D-glucosamin, D-Glucosamin, D-Fructose, L-Rhamnose; Cellobiose, Gentiobiose, Isomaltose, Isomaltulose, Lactulose, Laminaribiose, Maltose, Maltulose, Melibiose, Neohesperidose, Neotrehalose, Nigerose, Rutinose, Sophorose, Saccharose und Trehalose sowie deren Gemischen versetzt, dass das Löslichkeitsprodukt der noch in Lösung befindlichen Restmenge an Lactose überschritten wird,
(i) die zweite Menge ausgefallener alpha-Lactosekristalle von der Mutterlauge abtrennt, trocknet und
(j) die beiden Mengen an alpha-Lactosekristallen vereinigt.

### BEISPIELE

### VERGLEICHSBEISPIEL V1

Eine Lactose-Mutterlauge mit einem Gehalt von 89,5 Gew.-% in der Trockenmasse wurde mit einer Temperatur von etwa 70°C in einen auf 65 °C vorgeheizten Kristallisationstank gegeben, dort mit einer Geschwindigkeit von etwa 3 °K/h innerhalb von etwa 1 h auf 65 °C abgekühlt und für eine knappe Stunde gehalten, ehe die Lösung wiederum mit einer Geschwindigkeit von etwa 3 °K/h innerhalb von etwa 1 h auf 25 °C abgekühlt wurde. Dort wurde die Lösung weitere 3 h bei der Temperatur gehalten und dann mit einer Geschwindigkeit von 1 °K/h innerhalb 1 h bis auf 10 °C abgekühlt. Mit dem Beginn der Kristallabscheidung wurde die Lösung etwa 15 h bei dieser Temperatur gehalten, die ausgefallenen alpha-Lactosekristalle in einem Dekanter abgetrennt und auf einem Bandtrockner von anhaftender Feuchtigkeit befreit. Die zurückbleibende Mutterlauge wies einen Restgehalt von 34,7 Gew.-% Lactose auf. Das Temperatur/Zeit-Profil ist in **Abbildung 1** wiedergegeben.

### BEISPIEL 1

Die Lactose-Mutterlauge aus Vergleichsbeispiel V1 wurde mit einer Temperatur von etwa 95 °C in einen auf 65 °C vorgeheizten Kristallisationstank gegeben, dort mit einer Geschwindigkeit von etwa 3 °K/h innerhalb von etwa 1 h auf 65 °C abgekühlt dort für eine knappe Stunde gehalten, ehe die Lösung wiederum mit einer Geschwindigkeit von etwa 3 °K/h innerhalb von etwa 1 h auf 25 °C abgekühlt wurde. Dort wurde die Lösung weitere 3 h bei der Temperatur gehalten und dann mit einer Geschwindigkeit von etwa 3 °K/h wieder auf 37 °C erwärmt. Die Lösung wurde weitere 3 h bei dieser Temperatur gehalten und dann mit einer Geschwindigkeit von 1 °K/h innerhalb 1 h bis auf 10 °C abgekühlt. Mit dem Beginn der Kristallabscheidung wurde die Lösung etwa 12 h bei dieser Temperatur gehalten, die ausgefallenen alpha-Lactosekristalle in einem Dekanter abgetrennt und auf einem Bandtrockner von anhaftender Feuchtigkeit befreit. Die zurückbleibende Mutterlauge wies einen Restgehalt von 18,4 Gew.-% Lactose auf. Lactose auf. Das Temperatur/Zeit-Profil ist in **Abbildung 2** wiedergegeben.

### VERGLEICHSBEISPIEL V2

### Ohne Zugabe von Kohlenhydraten

Eine erste Lactose-Mutterlauge mit einem Gehalt von 89,5 Gew.-% in der Trockenmasse wurde mit einer Temperatur von etwa 95 °C in einen auf 65 °C vorgeheizten Kristallisationstank gegeben, dort mit einer Geschwindigkeit von etwa 3 °K/h innerhalb von etwa 1 h auf 65 °C abgekühlt und für eine knappe Stunde gehalten, ehe die Lösung wiederum mit einer Geschwindigkeit von etwa 3 °K/h innerhalb von etwa 13 h auf 25 °C abgekühlt wurde. Dort wurde die Lösung weitere 3 h bei der Temperatur gehalten und dann mit einer Geschwindigkeit von 1 °K/h innerhalb 1 h bis auf 10 °C abgekühlt. Mit dem Beginn der Kristallabscheidung wurde die Lösung etwa 15 h bei dieser Temperatur gehalten, die ausgefallenen alpha-Lactosekristalle in einem Dekanter abgetrennt und auf einem Bandtrockner von anhaftender Feuchtigkeit befreit. Die zurückbleibende zweite Mutterlauge wies einen Restgehalt von 34,7 Gew.-% Lactose auf.

### BEISPIELE 2 BIS 6

Zugabe von 0,1 bis 5 Gew.-% Glucose

Der Mutterlauge aus Beispiel 1 wurden 0,1 bis 5 Gew.-% Glucose zugesetzt. Die Lösung wurde homogenisiert und dann erneut wie oben beschrieben abgekühlt und die durch Absenkung des Löslichkeitsproduktes gewonnene zusätzliche Menge an Lactosekristallen abgetrennt. In **Tabelle 1** sind die Restgehalte an Lactose in der so erhaltenen dritten Mutterlauge in Abhängigkeit von der Zusatzmenge an Glucose wiedergegeben.

**Tabelle 1**

| Restgehalt Lactose in letzter Mutterlauge (Gew.-%) | | |
|---|---|---|
| **Beispiel** | **Glucosemenge** | **Restmenge Lactose in Mutterlauge** |
| Kontrolle | 0 | 18,4 |
| 2 | 0,1 | 9,7 |
| 3 | 0,5 | 8,8 |
| 4 | 1,0 | 7,6 |
| 5 | 2,0 | 5,2 |
| 6 | 5,0 | 5,2 |

### BEISPIELE 7 BIS 11

### Zugabe von 2 Gew.-% Kohlenhydrate

Der Mutterlauge aus Beispiel 1 wurden 2 Gew.-% verschiedener Kohlenhydrate zugesetzt. Die Lösung wurde homogenisiert, dann erneut wie oben beschrieben abgekühlt und die durch Absenkung des Löslichkeitsproduktes gewonnene zusätzliche Menge an Lactosekristallen abgetrennt. In **Tabelle 2** sind die Restgehalte an Lactose in der so erhaltenen dritten Mutterlauge in Abhängigkeit der Zusatzmenge an Kohlenhydrat wiedergegeben.

**Tabelle 2**

| Restgehalt Lactose in letzter Mutterlauge (Gew.-%) | | |
|---|---|---|
| **Beispiel** | **Kohlenhydrat** | **Restmenge Lactose in Mutterlauge** |
| Kontrolle | ohne | 18,4 |
| 7 | Glucose | 5,2 |
| 8 | Saccharose | 5,8 |
| 9 | Melasse | 6,2 |
| 10 | Zuckerrübensirup | 6,5 |
| 11 | Kornstärkesirup | 6,7 |

## Patentansprüche

1. Verfahren zur Verbesserung der Ausbeute bei der Herstellung von kristalliner alpha-Lactose, bei dem man
(a) eine wässrige Lactoselösung auf eine Temperatur von etwa 62 bis 67 °C einstellt,
(b) die Lösung dann bis auf etwa 20 bis 30 °C abkühlt,
(c) die Lösung bei dieser Temperatur für 0 bis 5 h hält,
(d) anschließend die Lösung wieder bis auf etwa 30 bis 45 °C erwärmt,
(e) die Lösung bei dieser Temperatur für 0 bis 5 h hält,
(f) danach die Lösung bis auf etwa 10 °C abkühlt und
(g) anschließend die ausgefallene erste Menge an alpha-Lactosekristallen von der Mutterlauge abtrennt,
(h) die Mutterlauge mit einer solchen Menge an Kohlenhydratverbindungen aus gewählt aus der Gruppe, die gebildet wird von L-Glycerinaldehyd, Dihydroxyaceton; D-Erythrose, D-Threose; D-Ribose, D- und L-Xylulose; D-Glucose, D-Mannose, D-Gulose, D-Idose, D-Galaktose, D-Glucronsäure, D-Galacturonsäure, N-Acetyl-D-glucosamin, D-Glucosamin, D-Fructose, L-Rhamnose; Cellobiose, Gentiobiose, Isomaltose, Isomaltulose, Lactulose, Laminaribiose, Maltose, Maltulose, Melibiose, Neohesperidose, Neotrehalose, Nigerose, Rutinose, Sophorose, Saccharose und Trehalose sowie deren Gemischen versetzt, dass das Löslichkeitsprodukt der noch in Lösung befindlichen Restmenge an Lactose überschritten wird,
(i) die zweite Menge ausgefallener alpha-Lactosekristalle von der Mutterlauge abtrennt, trocknet und
(j) die beiden Mengen an alpha-Lactosekristallen vereinigt.

2. Verfahren nach Anspruch 1, **dadurch gekennzeichnet, dass** man die Lactoselösung in Schritt (a) auf 63 bis 65 °C einstellt.

3. Verfahren nach den Ansprüchen 1 und/oder 2, **dadurch gekennzeichnet, dass** man die Lösung in Schritt (b) mit einer Geschwindigkeit von etwa 1 bis 5 °K/h abkühlt.

4. Verfahren nach mindestens einem der Ansprüche 1 bis 3, **dadurch gekennzeichnet, dass** man die Lösung in Schritt (b) bis auf bis auf 23 bis 26 °C abkühlt.

5. Verfahren nach mindestens einem der Ansprüche 1 bis 4, **dadurch gekennzeichnet, dass** man die Lösung in Schritt (c) für etwa 1 bis 3 h bei der Temperatur hält.

6. Verfahren nach mindestens einem der Ansprüche 1 bis 5, **dadurch gekennzeichnet, dass** man die Lösung im Schritt (d) auf etwa 32 bis 42 °C erwärmt.

7. Verfahren nach mindestens einem der Ansprüche 1 bis 6, **dadurch gekennzeichnet, dass** man die Lösung im Schritt (d) für etwa 1 bis 3 h bei dieser Temperatur hält.

8. Verfahren nach mindestens einem der Ansprüche 1 bis 7, **dadurch gekennzeichnet, dass** man die Lösung in Schritt (f) mit einer Geschwindigkeit von etwa 1 bis 5 °K/h abkühlt.

9. Verfahren nach mindestens einem der Ansprüche 1 bis 8, **dadurch gekennzeichnet, dass** das Temperaturprofil umfassend die Schritte (a) bis (g) innerhalb einer Zeitspanne von etwa 18 bis 24 h durchlaufen wird.

10. Verfahren nach mindestens einem der Ansprüche 1 bis 9, **dadurch gekennzeichnet, dass** die alpha-Lactosekristalle mit Hilfe eines Dekanters abgetrennt werden.

11. Verfahren nach mindestens einem der Ansprüche 1 bis 10, **dadurch gekennzeichnet, dass** man Glucose oder eine glucosereiche Kohlenhydratfraktion einsetzt.

12. Verfahren nach mindestens einem der Ansprüche 1 bis 11, **dadurch gekennzeichnet, dass** man die Kohlenhydratverbindungen der Mutterlauge in Mengen von bis zu 5 Gew.-% zusetzt.

13. Verfahren zur Herstellung von kristalliner alpha-Lactose, bei dem man
(i) Molke einem Trennverfahren unterwirft, bei dem eine proteinreiche und eine lactosereiche Fraktion erhalten wird,
(ii) die lactosereiche Fraktion gegebenenfalls nach Aufkonzentrierung Hauptstrom einer Demineralisierung unterwirft, bei der schwerlösliche Salze gefällt werden,
(iii) den demineralisierten Rückstand gegebenenfalls nach weiterer Aufkonzentrierung abkühlt, bis Lactose kristallin ausfällt,
(iv) die Lactosekristalle von der ersten Mutterlauge abtrennt und entwässert,
**dadurch gekennzeichnet, dass** man
(a) die erste Mutterlauge auf eine Temperatur von etwa 62 bis 67 °C einstellt,
(b) die Lösung dann bis auf etwa 20 bis 30 °C abkühlt,
(c) die Lösung bei dieser Temperatur für 0 bis 5 h hält,
(d) anschließend die Lösung wieder bis auf etwa 30 bis 45 °C erwärmt,
(e) die Lösung bei dieser Temperatur für 0 bis 5 h hält,
(f) danach die Lösung bis auf etwa 10 °C abkühlt und
(g) anschließend die ausgefallenen alpha-Lactosekristalle von der zweiten Mutterlauge abtrennt,
(h) die Mutterlauge mit einer solchen Menge an Kohlenhydratverbindungen aus gewählt aus der Gruppe, die gebildet wird von L-Glycerinaldehyd, Dihydroxyaceton; D-Erythrose, D-Threose; D-Ribose, D- und L-Xylulose; D-Glucose, D-Mannose, D-Gulose, D-Idose, D-Galaktose, D-Glucronsäure, D-Galacturonsäure, N-Acetyl-D-glucosamin, D-Glucosamin, D-Fructose, L-Rhamnose; Cellobiose, Gentiobiose, Isomaltose, Isomaltulose, Lactulose, Laminaribiose, Maltose, Maltulose, Melibiose, Neohesperidose, Neotrehalose, Nigerose, Rutinose, Sophorose, Saccharose und Trehalose sowie deren Gemischen versetzt, dass das Löslichkeitsprodukt der noch in Lösung befindlichen Restmenge an Lactose überschritten wird,
(i) die zweite Menge ausgefallener alpha-Lactosekristalle von der Mutterlauge abtrennt, trocknet und
(j) die beiden Mengen an alpha-Lactosekristallen vereinigt.

## Claims

1. A method for improving the yield during the production of crystalline alpha lactose, wherein
(a) an aqueous lactose solution is adjusted to a temperature of between about 62 and 67 °C,
(b) the solution is then cooled down to between about 20 and 30 °C,
(c) the solution is held at this temperature for a period of from 0 to 5 h,
(d) the solution is subsequently heated to between about 30 and 45 °C,
(e) the solution is held at this temperature for a period of from 0 to 5 h,
(f) the solution is then cooled down to about 10 °C, and
(g) subsequently, the precipitated first amount of alpha lactose crystals is separated from the mother liquor,
(h) an amount of a carbohydrate compound selected from the group consisting of L-glyceraldehyde, dihydroxyacetone, D-erythrose, D-threose; D-ribose, D- and L-xylulose; D-glucose, D-mannose, D-gulose, D-idose, D-galaktose, D-glucronsäure, D-galacturonsäure, N-acetyl-D-glucosamine, D-glucosamine, D-fructose, L-rhamnose; cellobiose, gentiobiose, isomaltose, isomaltulose, lactulose, laminaribiose, maltose, maltulose, melibiose, neohesperidose, neotrehalose, nigerose, rutinose, sophorose, saccharose, trehalose or their mixtures is added to the mother liquor such that the solubility product of the residual amount of lactose that is still soluble is exceeded,
(i) the second amount of precipitated alpha lactose crystals is separated from the mother liquor, dried, and
(j) both amounts of alpha lactose crystals are combined.

2. The method of claim 1, **characterized in that** the lactose solution under step (a) is adjusted to between 63 °C and 65 °C.

3. The method of claims 1 and/or 2, **characterized in that** the solution under step (b) is cooled down at a rate of from about 1 to 5 °K/h.

4. The method of at least one of claims 1 to 3, **characterized in that** the solution under step (b) is cooled down to between 23 and 26 °C.

5. The method of at least one of claims 1 to 4, **characterized in that** the solution under step (c) is held at this temperature for a period of about 1 to 3 h.

6. The method of at least one of claims 1 to 5, **characterized in that** the solution under step (d) is heated to between about 32 and 42 °C.

7. The method of at least one of claims 1 to 6, **characterized in that** the solution under step (d) is held at this temperature for a period of about 1 to 3 h.

8. The method of at least one of claims 1 to 7, **characterized in that** the solution under step (f) is cooled down at a rate of about 1 to 5 °K/h.

9. The method of at least one of claims 1 to 8, **characterized in that** the temperature profile comprising steps (a) to (g) is passed through within a period of about 18 to 24 h.

10. The method of at least one of claims 1 to 9, **characterized in that** the alpha lactose crystals are separated by means of a decanter.

11. The method of at least one of claims 1 to 10, **characterized in that** glucose or a carbohydrate fraction that is rich in glucose is used.

12. The method of at least one of claims 1 to 11, **characterized in that** carbohydrate compounds are added to the mother liquor in amounts of up to 5 % by weight.

13. A method for the production of crystalline alpha lactose, wherein
(i) whey is subjected to a separation process in which a protein-rich and a lactose-rich fraction is obtained,
(ii) the lactose-rich fraction is (optionally after concentrating the main flow) subjected to demineralisation in which slowly soluble salts are precipitated,
(ii) the demineralised residue is cooled down, optionally after further concentration, until lactose precipitates in crystalline form,
(iii) the lactose crystals are separated from the mother liquor and evaporated,
**characterized in that**
(a) the first mother liquor is adjusted to a temperature of between about 62 and 67 °C,
(b) the solution is then cooled down to between about 20 and 30 °C,
(c) the solution is held at this temperature for a period of from 0 to 5 h,
(d) subsequently, the solution is re-heated to between about 30 and 35 °C,
(e) the solution is held at this temperature for a period of from 0 to 5 h,
(f) then the solution is cooled to about 10 °C and
(g) subsequently, the precipitated alpha lactose crystals are separated from the second mother liquor,
(h) an amount of a carbohydrate compound selected from the group consisting of L-glyceraldehyde, dihydroxyacetone, D-erythrose, D-threose; D-ribose, D- and L-xylulose; D-glucose, D-mannose, D-gulose, D-idose, D-galaktose, D-glucronsäure, D-galacturonsäure, N-acetyl-D-glucosamine, D-glucosamine, D-fructose, L-rhamnose; cellobiose, gentiobiose, isomaltose, isomaltulose, lactulose, laminaribiose, maltose, maltulose, melibiose, neohesperidose, neotrehalose, nigerose, rutinose, sophorose, saccharose, trehalose or their mixtures is added to the second mother liquor such that the solubility product of the residual amount of lactose that is still soluble is exceeded,
(i) the second amount of precipitated alpha lactose crystals is separated from the mother liquor, dried, and
(j) both amounts of alpha lactose crystals are combined.

## Revendications

1. Procédé d'amélioration du rendement lors de la fabrication d'alpha-lactose cristallin, selon lequel
(a) une solution aqueuse de lactose est ajustée à une température d'environ 62 à 67 °C, puis
(b) la solution est refroidie à une température d'environ 20 à 30 °C,
(c) la solution est maintenue à cette température pendant 0 à 5 h, puis
(d) la solution est de nouveau portée à une température d'environ 30 à 45 °C,
(e) la solution est maintenue à cette température pendant 0 à 5 h, ensuite
(f) la solution est refroidie à environ 10 °C, et puis
(g) la première quantité précipitée de cristaux d'alpha-lactose est séparée de la liqueur mère,
(h) la liqueur mère est mélangée avec une quantité de composés glucidiques choisis dans le groupe constitué par le L-glycérine-aldéhyde, la dihydroxyacétone ; le D-érythrose, le D-thréose ; le D-ribose, le D- et le L-xylulose ; le D-glucose, le D-mannose, le D-gulose, le D-idose, le D-galactose, l'acide D-glucronique, l'acide D-galacturonique, la N-acétyl-D-glucosamine, la D-glucosamine, le D-fructose, le L-rhamnose ; le cellobiose, le gentiobiose, l'isomaltose, l'isomaltulose, le lactulose, le laminaribiose, le maltose, le maltulose, le mélibiose, le néohespéridose, le néotréhalose, le nigérose, le rutinose, le sophorose, la saccharose et le tréhalose, ainsi que leurs mélanges, telle que le produit de solubilité de la quantité résiduelle de lactose se trouvant encore en solution soit dépassé,
(i) la seconde quantité de cristaux d'alpha-lactose précipités est séparée de la liqueur mère, séchée, et
(j) les deux quantités de cristaux d'alpha-lactose sont réunies.

2. Procédé selon la revendication 1, **caractérisé en ce que** la solution de lactose est ajustée à une température de 63 à 65 °C à l'étape (a).

3. Procédé selon les revendications 1 et/ou 2, **caractérisé en ce que** la solution est refroidie à une vitesse d'environ 1 à 5 °K/h à l'étape (b).

4. Procédé selon au moins l'une quelconque des revendications 1 à 3, **caractérisé en ce que** la solution est refroidie à une température de 23 à 26 °C à l'étape (b)

5. Procédé selon au moins l'une quelconque des revendications 1 à 4, **caractérisé en ce que** la solution est maintenue à la température pendant environ 1 à 3 h à l'étape (c).

6. Procédé selon au moins l'une quelconque des revendications 1 à 5, **caractérisé en ce que** la solution est portée à une température d'environ 32 à 42 °C à l'étape (d).

7. Procédé selon au moins l'une quelconque des revendications 1 à 6, **caractérisé en ce que** la solution est maintenue à cette température pendant environ 1 à 3 h à l'étape (d).

8. Procédé selon au moins l'une quelconque des revendications 1 à 7, **caractérisé en ce que** la solution est refroidie à une vitesse d'environ 1 à 5 °K/h à l'étape (f).

9. Procédé selon au moins l'une quelconque des revendications 1 à 8, **caractérisé en ce que** le profil de température comprenant les étapes (a) à (g) est traversé en une durée d'environ 18 à 24 h.

10. Procédé selon au moins l'une quelconque des revendications 1 à 9, **caractérisé en ce que** les cristaux d'alpha-lactose sont séparés à l'aide d'un décanteur.

11. Procédé selon au moins l'une quelconque des revendications 1 à 10, **caractérisé en ce que** du glucose ou une fraction glucidique riche en glucose est utilisé.

12. Procédé selon au moins l'une quelconque des revendications 1 à 11, **caractérisé en ce que** les composés glucidiques sont ajoutés à la liqueur mère en quantités de jusqu'à 5 % en poids.

13. Procédé de fabrication d'alpha-lactose cristallin, selon lequel
(i) du lactosérum est soumis à un procédé de séparation, lors duquel une fraction riche en protéines et une fraction riche en lactose sont obtenues,
(ii) la fraction riche en lactose est soumise, éventuellement après une concentration du courant principal, à une déminéralisation lors de laquelle des sels difficilement solubles sont précipités,
(iii) le résidu déminéralisé est refroidi, éventuellement après une concentration supplémentaire, jusqu'à la précipitation de lactose cristallin,
(iv) les cristaux de lactose sont séparés de la première liqueur mère et déshydratés,
**caractérisé en ce que**
(a) la première liqueur mère est ajustée à une température d'environ 62 à 67 °C, puis
(b) la solution est refroidie à une température d'environ 20 à 30 °C,
(c) la solution est maintenue à cette température pendant 0 à 5 h, puis
(d) la solution est de nouveau portée à une température d'environ 30 à 45 °C,
(e) la solution est maintenue à cette température pendant 0 à 5 h, ensuite
(f) la solution est refroidie à environ 10 °C, et puis
(g) les cristaux d'alpha-lactose précipités sont séparés de la seconde liqueur mère,
(h) la liqueur mère est mélangée avec une quantité de composés glucidiques choisis dans le groupe constitué par le L-glycérine-aldéhyde, la dihydroxyacétone ; le D-érythrose, le D-thréose ; le D-ribose, le D- et le L-xylulose ; le D-glucose, le D-mannose, le D-gulose, le D-idose, le D-galactose, l'acide D-glucronique, l'acide D-galacturonique, la N-acétyl-D-glucosamine, la D-glucosamine, le D-fructose, le L-rhamnose ; le cellobiose, le gentiobiose, l'isomaltose, l'isomaltulose, le lactulose, le laminaribiose, le maltose, le maltulose, le mélibiose, le néohespéridose, le néotréhalose, le nigérose, le rutinose, le sophorose, la saccharose et le tréhalose, ainsi que leurs mélanges, telle que le produit de solubilité de la quantité résiduelle de lactose se trouvant encore en solution soit dépassé,
(i) la seconde quantité de cristaux d'alpha-lactose précipités est séparée de la liqueur mère, séchée, et
(j) les deux quantités de cristaux d'alpha-lactose sont réunies.
